# EUROPÄISCHE PATENTSCHRIFT

(11) **EP 0 895 783 B1**
(45) Veröffentlichungstag und Bekanntmachung des Hinweises auf die Patenterteilung: **12.05.2004**
(21) Anmeldenummer: 98113170.9
(22) Anmeldetag: 15.07.1998
(51) Int. Cl.: A61K 31/675

(54) **Verwendung von Carnitin zur Antagonisierung der durch Ifosfamid verursachten Schädigung des Tubulus**
Use of Carnitine for antagonising the tubular damage caused by Ifosfamide
Utilisation de Carnitine pour antagoniser les lésions tubulaires causées par l'Ifosfamide

(30) Priorität: 01.08.1997 DE 19733305
(43) Veröffentlichungstag der Anmeldung: 10.02.1999
(73) Patentinhaber: Baxter Healthcare SA, 8304 Wallisellen (CH)
(72) Erfinder: Nickel, Bernd, Dr., 64367 Mühltal (DE); Pohl, Joerg, Dr., 63128 Dietzenbach (DE); Nolte, Thomas, Dr., 33829 Borgholzhausen (DE); Hilgard, Peter, Dr., 60325 Frankfurt (DE); Engel, Jürgen, Prof., Dr., 63755 Alzenau (DE); Schlenzig, J. S., Dr., 60322 Frankfurt (DE)
(74) Vertreter: Bachmann, Jürgen, Dr.

(56) Entgegenhaltungen:
- EP-A- 0 468 245
- EP-A- 0 469 440
- BRUEGGEMANN ET AL: "Prevention of Tubular Damage of 4-OH-Ifosfamide or Chloroacetaldehyde by Different Thiols" ANNALS OF HEMATOLOGY, Bd. 73, Nr. S2, 1996, Seite A86 XP002098710
- SCHLENZIG ET AL: "L-Carnitine: A Way to Decrease Cellular Toxicity of Ifosfamide?" EUROPEAN JOURNAL OF PEDIATRICS, Bd. 154, Nr. 8, 1995, Seiten 686-690, XP002098711

## Beschreibung

Die Erfindung betrifft die Verwendung von Carnithin bzw. dessen Derivate zur Herstellung eines Arzneimittels zur Antagonisierung der durch Ifosfamid verursachten Schädigung des proximalen Tubulus der Niere in Patienten, die mit Ifosfamid behandelt werden, wobei die Antitumorwirkung von Ifosfamid durch die Verabreichung von Carnithin nicht aufgehoben oder abgeschwächt wird, zur Verwendung für die Krebstherapie mit verbesserter Verträglichkeit, insbesondere geringerer Nephrotoxizität.

Es ist bekannt und beschrieben, daß Ifosfamid bei Patienten bei der Behandlung von Krebsleiden Nebenwirkungen hervorruft. Diese äußern sich bei den mit Ifosfamid behandelten Patienten durch die Schädigung des proximalen Tubulus der Niere (Pediatr. Nephrol.1994,8:157-163; Renal Physiol. Biochem.1992 15:289-301 ibid. 1993, 16:285-298)

Aus verschiedenen Publikationen geht ferner die Coadministration von Oxaphosphorinanen wie Ifosfamid mit Mercaptoethansulfonat (Mesna) zur Krebsbehandlung hervor, wobei die urotoxische Wirkung gesenkt wird.

Vom Carnitin ist bekannt, daß es bei systemischen Carnitinmangel, Muskeldystrophie mit Lipidakkumulation eingesetzt wird. Es verbessert ferner die Belastungsfähigkkeit und die Regenerationsfähigkeit des Muskels ( Münch. med. Wschr. 138(1996) Nr. 23 S. 53

In weltweiten Studien konnte bei neurologischen Ausfällen und Hirnleistungsstörungen wie senile Demenz und Alzheimersche Erkrankung durch L-Carnitin einen Besserung erzielt werden.

Es wird weiter im Bereich der cardiovaskulären Erkrankungen in der Behandlung von akuter und chronischer Myocardischämie, Angina pectoris sowie Herzarrhythmie und Insuffizienz sowie bei chronische Uraemie bei Dialysepatienten eingesetzt.

Aus der EP 0 722 724 ist bekannt, L-Carnitin und seine Derivate zur Verminderung des toxischen Effektes von Cyclosporin A und anderen Immunsuppressiva zu verwenden.

Schließlich sind aus einer Arbeit von Schlenzig et.al. in Eur. J. Pediatr. (1995) 154:686-690 Versuche an Ratten bekannt L-Carnitin mit Ifosfamid zusammen zu verabreichen, wobei eine Verbesserung des klinischen Befundes (verminderte Lethargie ) beobachtet wurde und nur eine leichte Senkung der Intermediate des Tricarbonsäurecyclus im Urin. Es wird angedeutet, daß die Ergänzung mit L-Carnitin eine Verbesserung bei der Ifosfamid-Behandlung bringen könnte.

Ziel der vorliegenden Erfindung war, Substanzen zu charakterisieren, die in Kombination mit Ifosfamid die bekannten Nebenwirkungen ( Schädigung des proximalen Tubulus der Niere) antagonisieren. Dabei mußte sichergestellt werden, daß die Antitumorwirkung von Ifosfamid durch die Kombination mit dem Antidot nicht aufgehoben oder abgeschwächt wird und keine zusätzlichen Nebenwirkungen durch die Gabe der Kombination auftreten.

Es wurden entsprechend der gestellten Aufgabe vergleichende Untersuchungen nach alleiniger Gabe von Ifosfamid und in Kombination mit L-Carnitin bezüglich Einfluß auf die Nephrotoxizität an gesunden und tumortragenden Ratten untersucht.
Die Dosierungen für L-Carnitin wurde so gewählt, daß die Verbindung selbst keinerlei Nebenwirkungen bei den Tieren hervorruft.

Bei der alleinigen Gabe von Ifosfamid kommt es, wie zu erwarten war, zu einem deutlichen dosisabhängigen Schädigung des proximalen Tubulus der Nieren bei beiden Tiergruppen.

Diese spezifische Schädigung wird überraschender Weise erfindungsgemäß durch die gleichzeitige Gabe von L- Carnitin (2x 100 mg/kg i.v.) signifikant antagonisiert. ( Tab. 1).

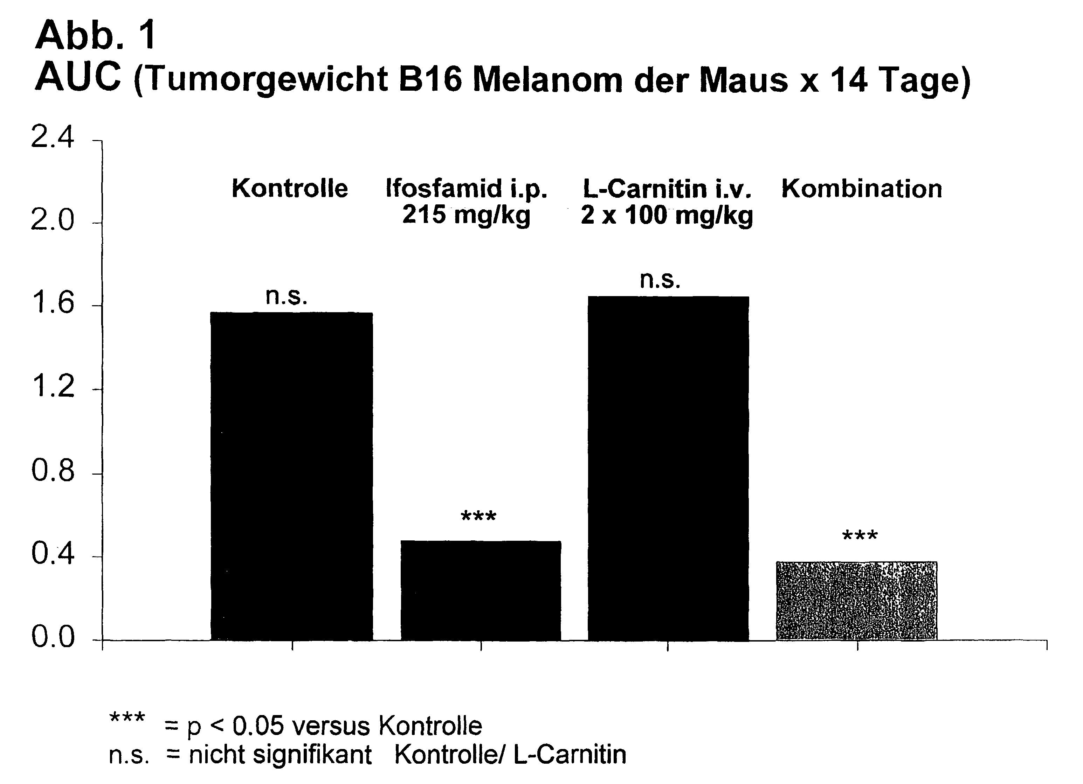

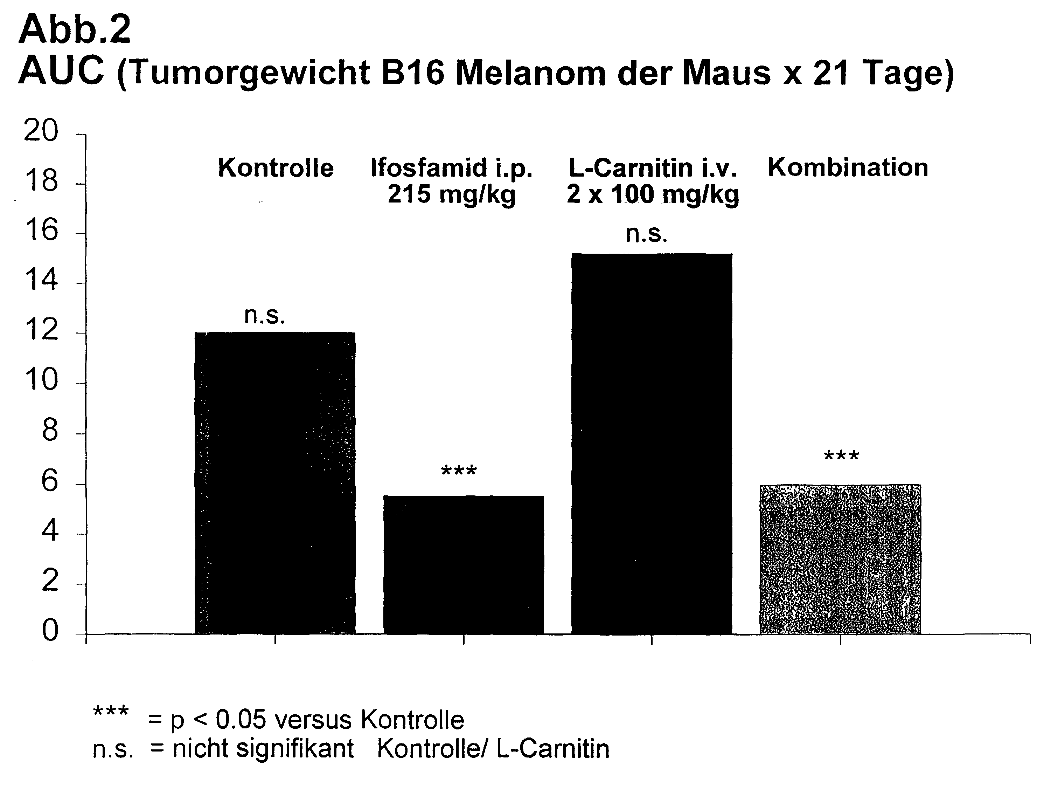

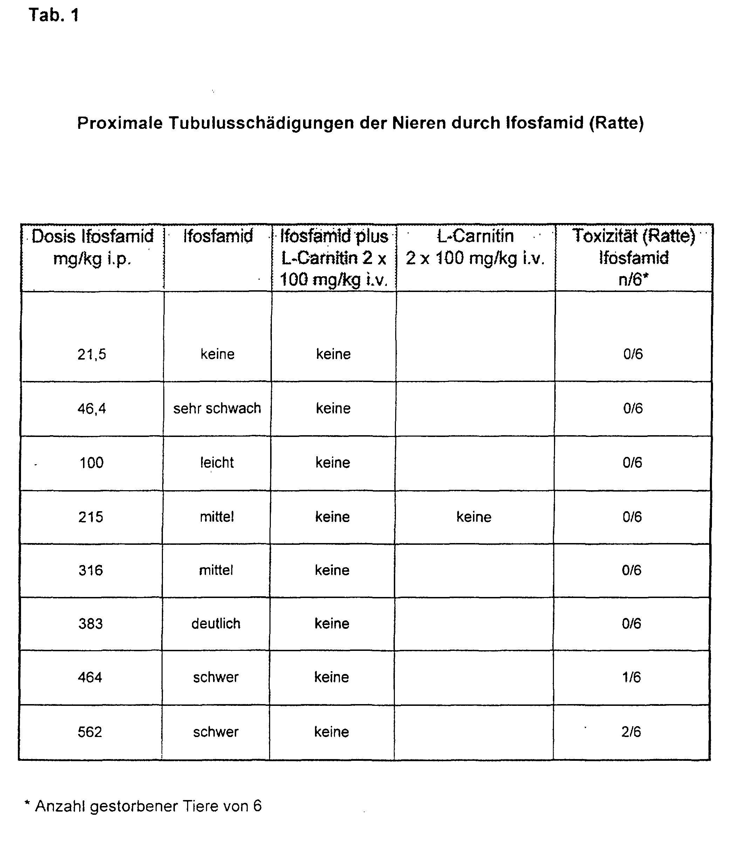

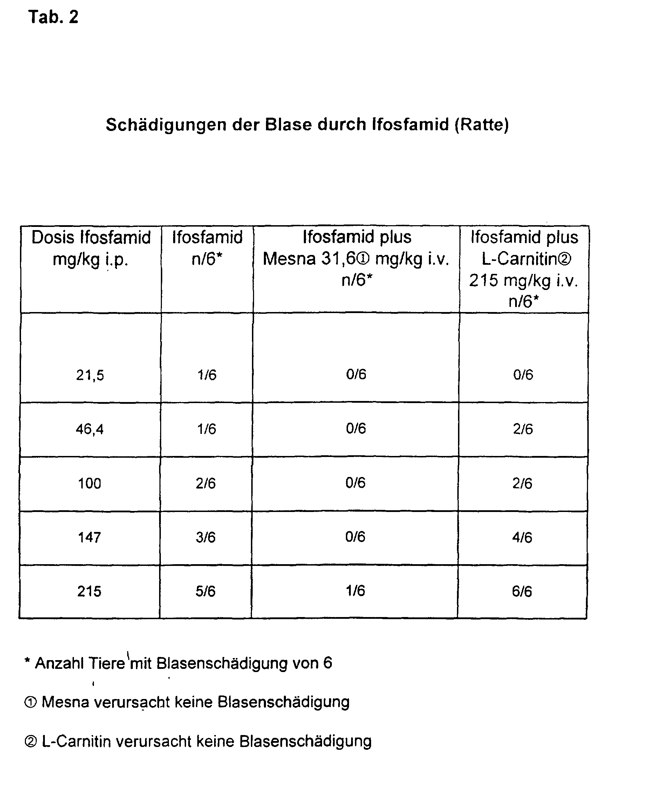

Die antitumor Wirkung von Ifosfamid wird in der Kombination mit L-Carnitin nicht beeinflußt (Abb. 1 und 2).

Gegenstand der Erfindung ist folglich die Verwendung von L-Carnitin in Form der freien Base oder als physiologisch verträgliches Salz wie L-Tartrat, Magnesium Citrat oder als Acetyl-L-Carnitin HCl zur Herstellung von Cytostatika mit Ifosfamid und zwar in fixer oder freier Kombination. Dabei liegen die appliziierten Dosen in den bekannten Größenordnungen d.h. beim Carnitin bzw. seinen Derivaten können pro Patient bis zu 5g mit der Ifosfamid-Dosis verabreicht werden. Bevorzugt wird jedoch ein Verhältnis von Ifosfamid zu Carnitin von 1 zu 10 bis 1 zu 20.

Ferner kann zur Vermeidung der bekannten toxischen Wirkung von Ifosfamid auf die Blase zusätzlich Mesna in der gleichen oder als getrennte Dosiseinheit in der an sich bekanntenn Dosis eingesetzt werden (Tab.2).

Damit wird ein komplexer Schutz der Niere und der Blase während der Tumorbehandlung mit Ifosfamid erzielt.

## Patentansprüche

1. Verwendung von Camithin zur Herstellung eines Arzneimittels zur Antagonisierung der durch Ifosfamid verursachten Schädigung des proximalen Tubulus der Niere in Patienten, die mit Ifosfamid behandelt werden, wobei die Antitumorwirkung von Ifosfamid durch die Verabreichung von Camithin nicht vermindert wird.

2. Verwendung von Camithin nach Anspruch 1, wobei das Camithin als L-Carnithin Base oder in Form physiologisch verträglicher Salze wie dem L-Tartrat, Magnesium Citrat oder als Acetyl-L-Camithin HCl eingesetzt wird.

3. Verwendung nach Anspruch 1 oder 2, wobei Camithin und Ifofamid in Form von Injektionslösungen verabreicht werden.

4. Verwendung nach einem der Ansprüche 1 bis 3, wobei Camithin in Form einer Trinklösung oder als Injektion oder Infusion gemeinsam oder getrennt von der Ifosfamidgabe verabreicht wird.

5. Verwendung nach einem der Ansprüche 1 bis 4, wobei das verabreichte Gewichtsverhältnis Ifosfamid zu Camithin 1 zu 10 bis 1 zu 20 beträgt.

6. Verwendung nach einem der Ansprüche 1 bis 5, wobei eine therapeutische Dosis Ifosfamid zusammen mit Camithin entweder in einer Dosiseinheit oder in getrennten Dosiseinheiten verabreicht wird.

7. Verwendung nach einem der Ansprüche 1 bis 6, wobei zusätzlich Mesna verabreicht wird.

8. Verwendung nach Anspruch 7, wobei eine therapeutische Dosis Ifosfamid zusammen mit Camithin und Mesna entweder in einer Dosiseinheit oder in getrennten Dosiseinheiten verabreicht wird.

9. Verwendung nach einem der Ansprüche 1 bis 8, wobei die Gabe von Ifosfamid und L-Camithin gleichzeitig erfolgt.

10. Verwendung nach einem der Ansprüche 1 bis 9, wobei L-Camithin und Ifosfamid in fixer oder freier Kombination verabreicht werden.

## Claims

1. Use of carnitine for the production of a medicament for antagonizing the damage caused by ifosfamide to the proximal tubule of the kidney in patients treated with ifosfamide, where the antitumour effect of ifosfamide is not reduced by the administration of carnitine.

2. Use of carnitine according to Claim 1, where the carnitine is employed as L-carnitine base or in the form of physiologically tolerated salts such as of L-tartrate, magnesium citrate or acetyl-L-carnitine HCl.

3. Use according to Claim 1 or 2, where carnitine and ifofamide are administered in the form of injection solutions.

4. Use according to any of Claims 1 to 3, where carnitine is administered together with or separately from the ifosfamide administration in the form of a drinking solution or as injection or infusion.

5. Use according to any of Claims 1 to 4, where the administered weight ratio of ifosfamide to carnitine is 1 : 10 to 1 : 20.

6. Use according to any of Claims 1 to 5, where a therapeutic dose of ifosfamide is administered together with carnitine either in one dose unit or in separate dose units.

7. Use according to any of Claims 1 to 6, where mesna is additionally administered.

8. Use according to Claim 7, where a therapeutic dose of ifosfamide is administered together with carnitine and mesna either in one dose unit or in separate dose units.

9. Use according to any of Claims 1 to 8, where the administration of ifosfamide and L-carnitine takes place simultaneously.

10. Use according to any of Claims 1 to 9, where L-carnitine and ifosfamide are administered in fixed or free combination.

## Revendications

1. Utilisation de la carnitine pour la préparation d'un médicament destiné à antagoniser l'endommagement causé par l'ifosfamide au tubule proximal du rein chez des patients traités par l'ifosfamide, l'effet antitumoral de l'ifosfamide n'étant pas réduit par l'administration de la carnitine.

2. Utilisation de la carnitine selon la revendication 1, la carnitine étant employée sous forme de la L-carnitine base ou sous forme de sels physiologiquement acceptables tels que du L-tartrate, du citrate de magnésium ou sous forme d'acétyl-L-carnitine HCl.

3. Utilisation selon la revendication 1 ou 2, la carnitine et l'ifosfamide étant administrés sous forme de solutions pour injection.

4. Utilisation selon l'une quelconque des revendications 1 à 3, la carnitine étant administrée conjointement avec ou séparément de l'administration d'ifosfamide sous forme d'une solution à boire ou sous forme d'une injection ou perfusion.

5. Utilisation selon l'une quelconque des revendications 1 à 4, le rapport pondéral entre l'ifosfamide et la carnitine administré est de 1 : 10 à 1 : 20.

6. Utilisation selon l'une quelconque des revendications 1 à 5, une dose thérapeutique d'ifosfamide étant administrée conjointement avec de la carnitine soit en une seule dose unitaire, soit en doses unitaires séparées.

7. Utilisation selon l'une quelconque des revendications 1 à 6, du mesna étant en outre administré.

8. Utilisation selon la revendication 7, une dose thérapeutique d'ifosfamide étant administrée conjointement avec de la carnitine et du mesna soit en une seule dose unitaire, soit en doses unitaires séparées.

9. Utilisation selon l'une quelconque des revendications 1 à 8, l'administration de l'ifosfamide et de la L-carnitine étant effectuée simultanément.

10. Utilisation selon l'une quelconque des revendications 1 à 9, la L-carnitine et l'ifosfamide étant administrés en combinaison fixe ou libre.
